(19) Europäisches Patentamt
European Patent Office
Office européen des brevets

(11) Numéro de publication: **0 100 257**
**B1**

(12) # FASCICULE DE BREVET EUROPEEN

(45) Date de publication du fascicule du brevet: **24.02.88**

(21) Numéro de dépôt: **83401386.4**

(22) Date de dépôt: **06.07.83**

(51) Int. Cl.$^4$: **C 07 C 87/28,** C 07 C 93/14, C 07 D 295/02, C 07 C 33/18, C 07 C 33/46, C 07 C 63/36, C 07 C 63/68, C 07 C 69/616, C 07 C 69/65, C 07 C 69/76, C 07 C 121/66

(54) **Nouveaux dérivés aminoalkyl naphtaléniques, leurs sels d'addition d'acide et le procédé de préparation ainsi que l'application en thérapeutique de ces dérivés et sels.**

(30) Priorité: **16.07.82 FR 8212493**

(43) Date de publication de la demande:
**08.02.84 Bulletin 84/06**

(45) Mention de la délivrance du brevet:
**24.02.88 Bulletin 88/08**

(84) Etats contractants désignés:
**BE CH DE FR GB IT LI LU NL SE**

(56) Documents cités:
**EP-A-0 035 363**
**FR-M- 8 291**
**US-A-3 663 608**

(73) Titulaire: **DELALANDE S.A.**
**32, rue Henri Regnault**
**F-92400 Courbevoie (FR)**

(72) Inventeur: **Schoofs, Alain-René**
**242, rue de la Croix-Nivert**
**F-75015 Paris (FR)**
Inventeur: **Bourgery, Guy**
**29, rue Denis Papin**
**F-92700 Colombes (FR)**
Inventeur: **Bucher, Bernard**
**23, boulevard de la République**
**F-92430 Marnes la Coquette (FR)**

(74) Mandataire: **Kedinger, Jean-Paul et al**
**c/o Cabinet Malemont 42, avenue du Président Wilson**
**F-75116 Paris (FR)**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 100 257 B1

Courier Press, Leamington Spa, England.

**Description**

La présente invention a pour objet de nouveaux dérivés aminoakyl naphtaléniques répondant plus précisément à la formule:

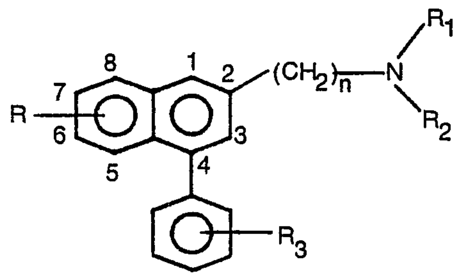

(I)

dans laquelle:

$R_1$ et $R_2$ sont identiques ou différents et représentent un atome d'hydrogène ou un groupe alkyle comportant de 1 à 4 atomes de carbone ou forment conjointement avec l'atome d'azote auquel ils sont liés, un radical hétérocyclique choisi parmi les suivants: pyrrolidino, pipéridino, hexaméthylèneimino, morpholino, pipérazino, méthyl-4 pipérazino;

n=1, 2 ou 3;

R représente un atome d'hydrogène ou d'halogène ou un groupe méthyle; et

$R_3$ représente un atome d'hydrogène ou d'halogène ou un groupe méthyle ou méthoxy.

Parmi ces dérivés, il convient de citer notamment les suivants:

dérivé pour lequel $NR_1R_2$ représente $NH_2$, n=2, R=H et $R_3$=H;

dérivés pour lesquels $NR_1R_2$ représente $NHCH_3$ et l'ensemble (n, R, $R_3$) prend l'une quelconque des valeurs suivantes: (1, H, H), (2, H, H) (2, H, H) (2, H, ortho-F);

dérivés pour lesquels $NR_1R_2$ représente $N(CH_3)_2$ et l'ensemble (n, R, $R_3$) prend l'une quelconque des valeurs suivantes: (1, H, H), (2, H, H) (2, H, ortho-F), (2, 8—Cl, H), (2, H, para-F), (2, H, méta-F), (2, H, ortho-$CH_3$), (2, H, méta-$CH_3$), (2, H, para-$CH_3$), (2, 6-Cl, H), (2, 7-Cl, H), (2, 6-F, H), (2, 6-$CH_3$, H), (3, H, H);

dérivés pour lesquels R=H, $R_3$=H, n=1 ou 2 et $NR_1R_2$ représente

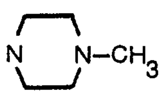 ; et

dérivés pour lesquels n=2, R=H, $R_3$=H et $NR_1R_2$ représente le radical pyrrolidino, pipéridino ou morpholino.

Des composés particulièrement préférés sont ceux pour lesquels l'ensemble (n, $NR_1R_2$, R, $R_3$) prend les valeurs (2, $NHCH_3$, H, ortho-F) et (2, $N(CH_3)_2$, H, ortho-F).

La présente invention concerne également les sels d'addition d'acide minéral (tel que l'acide chlorhydrique) ou organique (tel que l'acide maléïque), des dérivés de formule (I).

D'après US—A—3 663 608 et EP—A—0 035 365, on connait déjà des composés comportant un noyau phényle et une chaîne aminoalkyle portés respectivement par un groupe tétrahydronaphtalène ou indanique. Les composés connus diffèrent donc de ceux objet de la présente invention par la nature du groupe portant ce noyau phényle et cette chaîne aminoalkyle.

L'invention s'étend par ailleurs auprocédé de préparation des dérivés de formule (I) et de leurs sels.

Ainsi, l'invention s'étend auprocédé qui consiste à condenser les amines de formule:

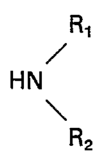

(II)

dans laquelle $R_1$ et $R_2$ ont la même signification que dans la formule (I), respectivement avec les composés de formule:

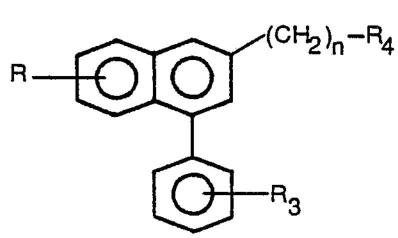

(III)

dans laquelle n, R et $R_3$ ont la même signification que dans la formule (I) et $R_4$ représente un groupement nucléophile bon partant; puis à éventuellement salifier les dérivés obtenus.

Le groupement nucléophile bon partant sera notamment constitué par un atome d'halogène ou par le groupe méthylsulfonyloxy ou p-tolylsulfonyloxy et la condensation sera effectuée de préférence dans un solvant aprotique.

Le composés de formule (III) pour lesquels $R_4$ représente un atome d'halogène peuvent être obtenus par action d'un oxyhalogénure de phosphore ou d'un pentahalogénure de phosphore, de préférence dans un solvant aprotique tel que le chlorure de méthylène, respectivement sur les composés de formule:

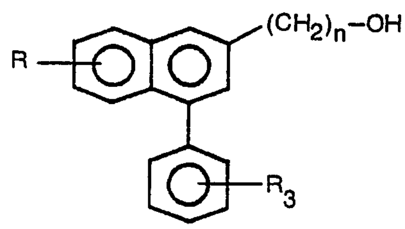

(IV)

dans laquelle R, $R_3$ et n ont la même signification que dans la formule (III).

Quant aux composés de formule (III) pour lesquels $R_4$ représente un groupement méthylsulfonyloxy ou para-tolylsulfonyloxy, ils peuvent être obtenus par action du chlorure de mésyle ou de tosyle, de préférence en milieu aprotique (tel que le chlorure de méthylène par exemple) et en présence d'une base (telle que la triéthylamine par exemple), respectivement sur les composés de formule (IV).

Les composés de formule (IV) sont obtenus par réduction par l'hydrure de lithium et d'aluminium (en milieu THF) des composés de formule:

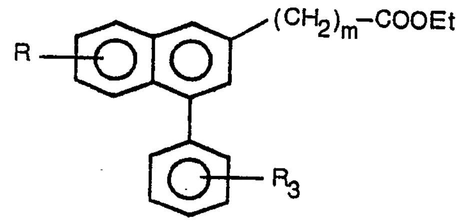

(V)

dans laquelle R et $R_3$ ont les mêmes significations que dans la formule (IV) et m prend la valeur 0, 1 ou 2.

Les composés de formule (V) pour lesquels m=O, sont obtenus par estérification par l'éthanol chlorhydrique, en milieu éthanolique des composés de formule:

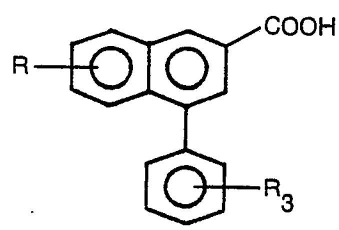

(VI)

dans laquelle R et $R_3$ ont les mêmes significations que dans la formule (V).

Les composés de formule (VI) sont obtenus par cyclisation aromatisante en milieu aprotique (tel que le benzène ou le chlorure de méthyle par exemple) et en présence d'un acide de Lewis (tel que le chlorure d'aluminium ou l'éthérate de trifluorure de bore par exemple), des composés de formule:

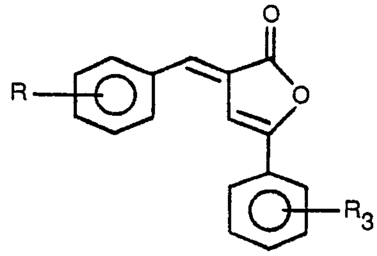

(VII)

dans laquelle R et $R_3$ ont les mêmes significations que dans la formule (VI).

Les composés de formule (VII) sont, quant à eux, obtenus par condensation dans l'anhydride acétique et en présence d'acétate de sodium, des composés de formule:

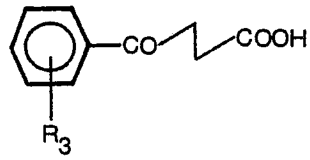

(VIII)

dans laquelle $R_3$ a les mêmes significations que dans la formule (VII), respectivement avec les aldéhydes aromatiques de formule:

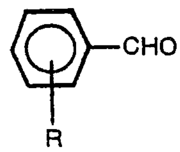

(IX)

dans laquelle R a les mêmes significations que dans la formule (VII).

Les composés de formule (V) pour lesquels m=1 ou 2, sont obtenus par estérification par l'éthanol chlorhydrique en milieu éthanolique des nitriles de formule:

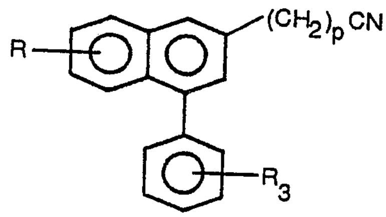

(X)

dans laquelle R et $R_3$ ont les mêmes significations que dans la formule (V) et p=1 ou 2.

Les composés de formule (X) sont obtenus par action d'un cyanure alcalin (tel que le cyanure de sodium par exemple) respectivement sur les composés de formule (III) pour lesquels n prend les valeurs 1 ou 2, la réaction étant effectuée en solution dans le DMSO ou le DMF.

Dans les cas où dans la formule (I), n prend la valeur 2 ou 3 et

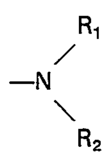

représente le groupe amino ($-NH_2$), les composés (I) correspondants peuvent également être avantageusement obtenus par réduction, de préférence catalytique, en présence de nickel de Raney des composés de formule (X) correspondants.

Les composés (VIII) sont soit déjà connus, soit obtenus selon les méthodes classiques de la littérature. Ainsi:

dans le cas où dans la formule (VIII), $R_3$ représente l'atome de fluor en position ortho ou méta ou le groupe méthyle en position méta, les composés (VIII) correspondants sont obtenus par hydrolyse acide, de préférence par l'acide chlorhydrique 6 N, des composés de formule:

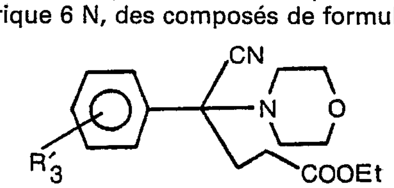

(XI)

dans laquelle $R'_3$ représente un atome de fluor en position 2 ou 3 ou le groupe méta-méthyle, ces derniers étant obtenus par condensation de l'acrylate d'éthyle, en présence d'une base (de préférence l'éthylate de sodium) en milieu THF — alcool, respectivement sur les composés de formule:

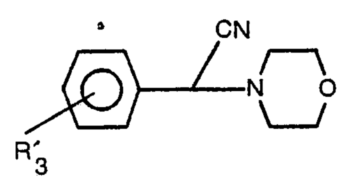

(XII)

dans laquelle $R'_3$ a les même significations que dans la formule (XI), eux-mêmes obtenus par une réaction dite de "STRECKER" entre le 2-(ou 3-) fluorbenzaldéhyde ou le 3-méthylbenzaldéhyde, la morpholine et un cyanure alcalin (de préférence le cyanure de potassium) en présence d'acide paratoluène sulfonique et en milieu aqueux; et

dans le cas où dans la formule (VIII), $R_3$ représente le groupe méthyle en position ortho, le composé (VIII) correspondant est obtenu par hydrolyse acide (de préférence par HC16N) du composé de formule:

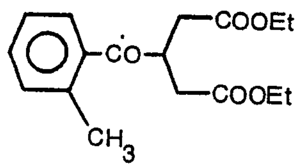

(XIII)

obtenu par condensation du bromoacétate d'éthyle, en milieu éther éthylique, en présence d'hydrure de sodium sur le composé de formule:

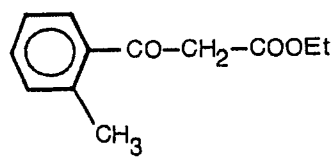

(XIV)

lui-même obtenu par condensation du monoester éthylique de l'acide malonique (HOOC 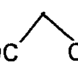 COOEt), dans le THF et en présence de butyl lithium, sur le composé de formule:

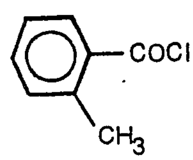

(XV)

Les sels d'addition d'acide des dérivés de formule (I) peuvent être obtenus par simple réaction d'un acide avec les dérivés de formule (I), dans des solvants appropriés.

Les préparations suivantes sont données à titre d'exemples pour illustrer l'invention.

Exemple 1

Chlorhydrate de N,N-diméthylaminométhyl-2-phényl-4- naphtalène (I) *Numéro de code:* 2

On porte à 50°C, en tube scellé, pendant 12 heures, un mélange de 6,4 g de mésyloxyméthyl-2 phényl-4 naphtalène (III) et de 36 cm3 d'une solution préparée à partir de 100 g de N,N-diméthylamine dans 800 cm3 de benzène, dans 100 cm3 de benzène.

Puis on évapore le solvant, reprend le résidu dans le chlorure de méthylène, lave à l'eau, extrait par une solution d'acide chlorhydrique 1 N, lave au chlorure de méthylène, basifie à l'aide de soude concentrée, extrait au chlorure de méthyène, lave à l'eau, sèche sur sulfate de soude, filtre et évapore le filtrat. On obtient 6,6 g (Rendement ~ 100%) d'une huile qui correspond au composé désiré sous forme de base. On dissout cette huile dans 100 ml d'alcool isopropylique et on ajoute 8,5 ml d'une solution 3,9 NB diéthanol chlorhydrique. On dilue par 250 ml d'éther et filtre le précipité obtenu. On isole ainsi 2,9 g (Rendement: 37%) du produit attendu, dont un certain nombre de données physico-chimiques sont rassemblées dans le tableau I ci-après.

Par le même procédé, mais à partir des réactifs correspondants, on obtient les composés de formule (I) de numéros de code 1 et 3 à 23 figurant dans le tableau I ci-après.

Exemple 2

Mésyloxyméthyl-2 phényl-4 naphtalène (III)

A une solution refroidie à — 10°C de 16,9 g d'hydroxyméthyl-2 phényl-4 naphtalène (IV) dans 250 ml de chlorure de méthylène, on ajoute lentement 25,2 ml de triéthylamine, puis 11,2 ml de chlorure de mésyle. On laisse à 0°C pendant 30 minutes, puis on dilue par de l'eau et de la glace, décante, lave à l'eau jusqu'à pH ~7, séche sur sulfate de sodium, filtre et évapore le filtrat. On obtient 20,8 g (Rendement: 92,30%) d'une huile brute (une seule tâche en C.C.M.) qui est employée pour la synthèse des composés de formule (I) correspondants selon l'exemple 1.

Formule brute: $C_{18}H_{16}O_3S$

Poids moléculaire: 312,37

Par le même procédé, mais à partir des réactifs correspondants, on obtient les autres composés de formule (III), nécessaires à la synthèse des composés de formule (I).

Exemple 3

Hydroxyméthyl-2 phényl-4 naphtalène (IV) *Numéro de code:* 1A

a 250 ml de THF refroidis à 0°C, on ajoute 3,7 g d'hydrure double de lithium et d'aluminium, puis lentement 27,1 g d'éthoxycarbonyl-2 phényl-4 naphtalène (V). On laisse 2 heures à 0°C, sous agitation, puis on hydrolyse à l'aide de sulfate de sodium humide, dilue par 100 ml d'éther, filtre, évapore la filtrat et chromatographie le résidu sur une colonne de silice (chromatographie liquide à moyenne pression, éluant: mélange heptane 60% — acétate d'éthyle 40%). On isole ainsi 16,8 g (Rendement: 73%) du produit attendu, après cristallisation dans l'éther de pétrole et recristallisation dans l'éther isopropylique.

Par le même procédé, mais à partir des réactifs correspondants, on obtient les composés de formule (IV) nécessaires à la synthèse des composés de formule (III) et notamment les composés de numéros de code: 1B, 6A, 6B, 7A, 7B, 8A, 8B, 14A, 14B, 15A, 15B, 16A, 16B, 17A, 17B, 18A, 18B, 19A, 19B, 20A, 20B, 21A, 21B et 1G figurant dans le tableau II ci-après.

Exemple 4

Ethoxycarbonyl-2 phényl-4 naphtalène (V)
*Numéro de code:* 1D

A une solution de 24,4 g d'acide (phényl-4 naphtalényl-2) carboxylique (VI) dans 350 ml d'éthanol, on ajoute 30 ml d'éthanol chlorhydrique 4,3 N. Puis on porte 7 heures au reflux, évapore le solvant, reprend le résidu dans le chlorure de méthylène, lave à l'aide d'une solution d'acide chlorhydrique 1 N, puis à l'eau, puis à l'aide d'une solution de soude 1 N, puis à l'eau. On sèche sur sulfate de sodium, filtre et évapore le filtrat. On obtient 27 g (Rendement ~ 100%) d'une huile brute qui correspond au composé attendu, unique en C.C.M. et qui est investi immédiatement dans le synthèse du composé de formule (IV) correspondant selon l'exemple 3.

Par le même procédé, mais à partir des réactifs correspondants, on obtient les composés de formule (V) pour lesquels m=0, de numéros de code 6D, 7D, 8D, 14D, 16D, 18D, 19D, 20D et 21D nécessaires à la synthèse des composés de formule (IV), ainsi que les composés de formule (V) pour lesquels m=1 ou 2, mais à partir des composés de formule (X) et notamment les composés de numéros de code 1E, 6E, 7E, 8E, 14E, 15E, 16E, 17E, 18E, 19E, 20E et 21E figurant dans le tableau II ci-après.

Exemple 5

Acide (phényl-4 naphtalènyl-2) carboxylique (VI)
*Numéro de code:* 1C

A un mélange de 24,7 g de chlorure d'aluminium dans 250 ml de benzène refroidi à 10°C, on ajoute, après une heure, 15,3 g d'α-benzylidène-α-phényl-$\Delta^{\beta\gamma}$-butènolide (VII), en maintenant la température entre 10 et 20°C pendant l'addition. Puis on laisse 3 heures à température ambiante, ajoute de l'acide chlorhydrique 1 N, extrait à l'éther, lave à l'eau, sèche sur sulfate de sodium, filtre, évapore le filtrat et cristallise le résidu dans l'éther isopropylique. On isole 8,5 g (Rendement: 55%) du produit attendu.

Par le même procédé, mais à partir des réactifs correspondants, on obtient les composés de formule (VI) nécessaires à la synthèse des composés de formule (V) correspondants, et notamment ceux de numéros de code 6C, 7C, 8C, 14C, 15C, 16C, 17C, 18C, 19C, 20C et 21C figurant dans le tableau II ci-après.

Exemple 6

α-Benzylidène-γ-phényl, —$\Delta^{\beta\gamma}$-butènolide (VII)

On chauffe jusqu'à obtenir une solution, puis on porte à 100°C pendant 4 heures un mélange de 10, 2 ml de benzaldéhyde (IX), de 17,8 g d'acide β-benzoyl-propionique (VIII) et de 8,2 g d'acétate de sodium dans 32 ml d'anhydride acétique.

Puis on laisse refroidir, ajoute de l'eau, filtre le précipité obtenu, le lave à l'eau sur le filtre et le recristallise dans l'alcool. On obtient ainsi 15,3 g (Rendement: 62%) du produit attendu.

Point de fusion: 145°C

Formule brute: $C_{17}H_{11}O_2$

Poids moléculaire: 247,26

Par le même procédé, mais à partir des réactifs correspondants, on obtient les composés de formule (VII) nécessaires à la synthèse des composés de formule (VI).

Exemple 7

Cyanométhyl-2-phényl-4 naphtalène (X)
*Numéro de code:* 1F

On agite pendant 12 heures à température ambiante un mélange de 28,5 g de mésyloxyméthyl-2 phényl-4 naphtalène (III) et de 17,8 g de cyanure de sodium dans 300 ml de DMSO. Puis on ajoute de l'eau et de la glace, extrait au chlorure de méthylène, lave à l'eau (jusqu'à pH ≃ 7), sèche sur sulfate de magnésium, filtre, évapore le filtrat. On obtient ainsi 22 g (Rendement ~ 98%) du produit attendu qui se présente sous la forme d'une huile.

Par le même procédé, mais à partir des réactifs correspondants, on obtient les composés de formule (X) nécessaires à la synthèse des composés de formule (V) pour lesquels m=1 ou 2 et notamment ceux de numéros de code 6F, 7F, 8F, 14F, 15F, 16F, 17F, 18F, 19F et 20F figurant dans le tableau II.

Exemple 8

Acide β-orthofluorobenzoyl propionique (VIII)

*1er stade:* α-morpholino fluoro-2 benzonitrile (XII)

On ajoute à 0°C, 200 g d'orthofluorobenzaldéhyde, lentement à 306 g d'acide paratoluène sulfonique. Puis en 1 heure, on ajoute 280 g de morpholine puis en 15 minutes une solution de 105 g de cyanure de potassium dans 170 ml d'eau. Puis on porte au reflux pendant 1 heure, jette la solution dans 3 litres d'une solution à 10% de bicarbonate de soude, extrait au chlorure de méthylène, lave à l'aide d'une solution à 10% de bicarbonate de soude, puis à l'eau, sèche sur sulfate de magnèsium, filtre et évapore le filtrat. On obtient ainsi 350 g (Rendement: 98%) du produit attendu.

Point de fusion: 76°C

Formule brute: $C_{12}H_{13}FN_2O$

Poids moléculaire: 220,24

Par le même procédé, mais à partir des réactifs correspondants, on obtient:

l'α-morphilino fluoro-3 benzonitrile:

Point de fusion: 78°C

Formule brute: $C_{12}H_{13}FN_2O$

Poids moléculaire: 220,24

l'α-morpholino méthyl-3 benzonitrile (liquide):

Formule brute: $C_{13}H_{16}N_2O$

Poids moléculaire: 216,27

Spectre de RMN (δ ppm/$CDCl_3$) = 7,2, m (4 protons aromatiques)

4,7, s (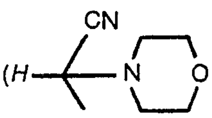)

3, 7 et 2,5 m (protons morpholiniques)

3, 95, s ($C\underline{H}_3$)

*2ème stade:* cyano-4 morpholino-4 orthofluorophényl-4 butyrate d'éthyle (XI)

A 450 ml d'éthanol refroidis à 0°C, on ajoute par petits morceaux 9,3 g de sodium, on porte à 40°C, puis la dissolution du sodium terminée, on refroidit à 0°C et ajoute en 2 heures et 15 minutes une solution de 200 g de composé (XII) obtenu au stade précédent, dans 800 ml de THF. Puis on ajoute une solution de 135 ml d'acrylate d'éthyle dans 100 ml de THF en 1 heure. On laisse 72 heures à température ambiante et évapore les solvants; on isole ainsi une huile rougeâtre (Rendement ~ 100%) qui est investie immédiatement dans le stade suivant.

*3ème stade:* Acide β-orthofluorobenzoyl propionique (VIII)

On porte au reflux, pendant 12 heures, 289 g du composé (XI) obtenu au stade précédent, dans 500 ml d'acide chlorhydrique 6 N. Puis on basifie à l'aide de soude concentrée, lave à l'éther, acidifie à l'aide d'acide chlorhydrique concentré, extrait au chlorure de méthylène, sèche sur sulfate de magnésium, filtre, évapore le filtrat et cristallise la pâte obtenue dans un mélange d'heptane (70%) et d'acétate d'éthyle (30%). On obtient ainsi 82 g (Rendement ~ 44%) du produit attendu.

Point de fusion: 98°C

Formule brute: $C_{10}H_{19}FO_3$

Poids moléculaire: 196, 17

*Spectre de RMN* (DMSO) δ ppm = 7, 1 à 7, 9, m (4 protons aromatiques); 14, 3, m ($COO\underline{H}$); 3, 2, m ($C\underline{H}_2$—COOH); 2, 6, t (J=7 Hz) (—CO—$C\underline{H}_2$)

*Spectre IR* (KBr): 1680 $cm^{-1}$; bande —CO—; 1720 $cm^{-1}$; bande —COOH

Par le même procédé, mais à partir des réactifs correspondants, on obtient:

l'acide β-métafluorobenzoyl propionique:

Point de fusion: 99°C

Formule brute: $C_{10}H_9FO_3$

Poids moléculaire: 196, 17

l'acide β-métaméthylbenzoyl propionique:

Point de fusion: 113°C

Formule brute: $C_{11}H_{12}O_3$

Poids moléculaire: 192, 21

Exemple 9

Chlorhydrate de l'(amino-2) éthyl-2 phényl-4 naphtalène (I)

*Numéro de code:* 9

A 10 g de compose 1F, décrit à l'exemple 7, dans 50 ml de méthanol, on ajoute 600 mg de nickel de Raney. On porte le mélange à 60°C puis on ajoute goutte à goutte en l'espace de 6 heures, 40 ml d'hydrate

d'hydrazine. Puis on filtre, évapore le filtrat, reprend le résidu dans l'eau, extrait à l'éther éthylique, puis extrait la phase organique à l'aide d'une solution d'acide chlorhydrique 1N, basifie à l'aide de NaOH concentrée, extrait au chlorure de méthylène, sèche sur sulfate de sodium (ou de magnésium), filtre et évapore le filtrat. Le résidu est dissous dans l'éther éthylique et on ajoute de l'éthanol chlorhydrique ≃ 6N, filtre le précipité obtenu et le recristallise dans un mélange d'éthanol et d'éther éthylique. On obtient ainsi le produit attendu.

Exemple 10

acide β-orthométhylbenzoyl propionique (VIII)

*1er stade:* ester éthylique de l'acide orthométhylbenzoyl acétique (XIV)

A une solution de 77 g de monoester éthylique de l'acide malonique dans 950 ml de THF, refroidie — à 70°C et sous atmosphère d'azote, on ajoute lentement en 2 heures 30 minutes 750 ml d'une solution 1,55 M de butyl lithium dans l'hexane. Puis on laisse revenir à — 5°C, laisse à cette température pendant 1 heurs 30 minutes, puis refoidit de nouveau à — 65°C et ejoute lentement en 35 minutes une solution de 90 g du chlorure de l'acide orthométhylbenzoïque (XV) dans 250 ml de THF. On laisse 1 heure à — 65°C, puis on verse le milieu réactionnel sur 2 litres d'eau glacée et 500 ml d'HCl 2N, extrait à l'éther, lave la solution éthérée avec une solution de bicarbonate de sodium, puis lave à l'eau, sèche sur sulfate de magnésium (ou de sodium), filtre et évapore le filtrat. L'huile obtenu est distillée ($Eb_{0,05\ mm/Hg}$ = 100°C). On obtient ainsi 78 g (rendement: 65%) du produit attendu.

*2ème stade:* diester éthylique de l'acide α-orthométhylbenzoyl succinique (XIII)

A 12,5 g d'hydrure de sodium à 80% on ajoute 400 ml d'éther éthylique, on refroidit à 0°C et on ajoute en 1 heure 15 minutes une solution de 78 g du composé (XIV) précédent dans 150 ml d'éther éthylique. On laisse 30 minutes à 0°C, puis on ajoute en 45 minutes, 52,5 ml de bromoacétate d'éthyle. On laisse 27 heures à température ambiante, puis on verse le mélange dans 700 ml d'eau glacée, décante, lave la phase organique à l'eau, sèche sur sulfate de magnésium (ou de sodium), filtre et évapore le filtrat. On obtient 110 g (rendement ≃100%) de produit brut, unique en C.C.M., qui est employé tel quel dans le stade suivant.

*3ème stade:* acide β-orthométhylbenzoyl propionique

On porte au reflux pendant 16 heures un mélange de 110 g de composé (XIII) précédent dans 650 ml d'acide chlorhydrique 6N. Puis on ajoute 2 litres d'eau glacée, puis 350 ml de potasse concentrée. On lave par de l'éther éthylique, puis acidifie par de l'acide chlorhydrique concentré, extrait au chlorure de méthylène, lave à l'eau, sèche sur sulfate de sodium ou de magnésium, filtre et évapore le filtrat. On obtient ainsi 40,5 g (rendement: 56%) du produit attendu.

. Point de fusion: 99°C
. Formule brute: $C_{11}H_{12}O_3$
. Poids moléculaire: 192,21

TABLEAU I

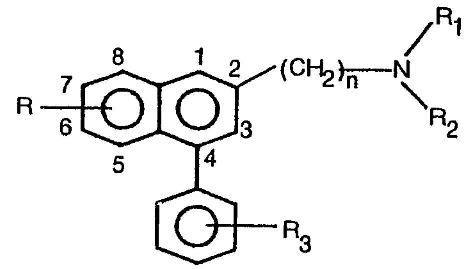

(I)

| Numéro de Code | 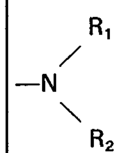 | n | R | $R_3$ | Forme | Formule brute | Poids moléculaire | Point de fusion (°C) | ANALYSE ELEMENTAIRE | | | |
|---|---|---|---|---|---|---|---|---|---|---|---|---|
| | | | | | | | | | % | C | H | N |
| 1 | 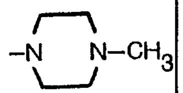 | 1 | H | H | diHCl +7% $H_2O$ | $C_{22}H_{26}N_2Cl_2$ +7% $H_2O$ | 418,66 | 222 | Cal. | 63.11 | 7,04 | 6,70 |
| | | | | | | | | | Tr. | 62,86 | 6,42 | 6,75 |
| 2 | 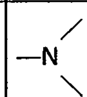 | " | " | " | HCl+5,3% $H_2O$ | $C_{19}H_{20}NCl$ +5,3% $H_2O$ | 314,48 | 127 | Cal. | 72,02 | 7,03 | 4,42 |
| | | | | | | | | | Tr. | 71,78 | 7,06 | 4,50 |
| 3 | —$NHCH_3$ | " | " | " | Maléate | $C_{22}H_{21}NO_4$ | 363,40 | 153 | Cal. | 72,71 | 5,83 | 3,85 |
| | | | | | | | | | Tr. | 72,67 | 6,02 | 3,84 |
| 4 | 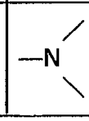 | 2 | " | " | HCl | $C_{20}H_{22}ClN$ | 311,84 | 202 | Cal. | 77,03 | 7,11 | 4,49 |
| | | | | | | | | | Tr. | 76,86 | 7,33 | 4,71 |

TABLEAU I (Suite)

| Numéro de Code | 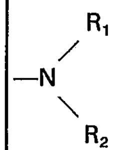 | n | R | $R_3$ | Forme | Formule brute | Poids molé-culaire | Point de fusion (°C) | ANALYSE ELEMENTAIRE % | C | H | N |
|---|---|---|---|---|---|---|---|---|---|---|---|---|
| 5 | 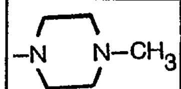 | 2 | H | H | diHCl | $C_{23}H_{28}Cl_2N_2$ | 403,38 | 182 | Cal. | 68,47 | 7,19 | 7,04 |
| | | | | | | | | | Tr. | 68,48 | 7,00 | 6,95 |
| 6 | 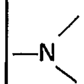 | " | " | 2-F | HCl+1,2% $H_2O$ | $C_{20}H_{21}FClN$ +1,2% $H_2O$ | 333,57 | 198 | Cal. | 72,00 | 6,48 | 4,20 |
| | | | | | | | | | Tr. | 72,24 | 6,63 | 4,26 |
| 7 | " | " | 8-Cl | H | HCl | $C_{20}H_{21}Cl_2N$ | 346,29 | 218 | Cal. | 69,36 | 6,11 | 4,05 |
| | | | | | | | | | Tr. | 69,06 | 6,25 | 4,01 |
| 8 | " | " | H | 4-F | " | $C_{20}H_{21}FClN$ | 329,83 | 221 | Cal. | 72,82 | 6,42 | 4,25 |
| | | | | | | | | | Tr. | 72,91 | 6,68 | 4,33 |

TABLEAU I (Suite)

| Numéro de Code | 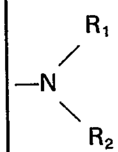 | n | R | $R_3$ | Forme | Formule brute | Poids molé-culaire | Point de fusion (°C) | ANALYSE ELEMENTAIRE % | C | H | N |
|---|---|---|---|---|---|---|---|---|---|---|---|---|
| 9 | $-NH_2$ | 2 | H | H | HCl | $C_{18}H_{18}ClN$ | 283,79 | 250 | Cal. | 76,18 | 6,39 | 4,94 |
| | | | | | | | | | Tr. | 76,20 | 6,08 | 4,79 |
| 10 | $-NHCH_3$ | " | " | " | " | $C_{19}H_{20}ClN$ | 297,81 | 230 | Cal. | 76,62 | 6,77 | 4,70 |
| | | | | | | | | | Tr. | 76,34 | 7,08 | 4,39 |
| 11 | 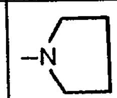 | " | " | " | " | $C_{22}H_{24}ClN$ | 337,88 | 215 | Cal. | 78,20 | 7,16 | 4,15 |
| | | | | | | | | | Tr. | 78,03 | 7,21 | 4,07 |
| 12 | 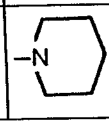 | " | " | " | " | $C_{23}H_{26}ClN$ | 351,90 | 248 | Cal. | 78,50 | 7,45 | 3,98 |
| | | | | | | | | | Tr. | 78,62 | 7,56 | 4,15 |

TABLEAU I (Suite)

| Numéro de Code | 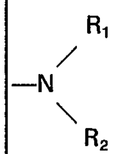 | n | R | $R_3$ | Forme | Formule brute | Poids molé-culaire | Point de fusion (°C) | ANALYSE ELEMENTAIRE % | C | H | N |
|---|---|---|---|---|---|---|---|---|---|---|---|---|
| 13 | 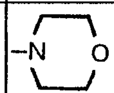 | 2 | H | H | HCl | $C_{22}H_{24}ClNO$ | 353,88 | 230 | Cal. | 74,66 | 6,84 | 3,96 |
| | | | | | | | | | Tr. | 74,32 | 6,73 | 3,60 |
| 14 | 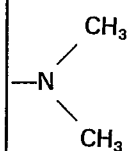 | " | " | 3-F | HCl+0,5% $H_2O$ | $C_{20}H_{21}ClFN$ +0,5% $H_2O$ | 331,49 | 218 | Cal. | 72,46 | 6,45 | 4,23 |
| | | | | | | | | | Tr. | 72,40 | 6,69 | 4,14 |
| 15 | " | " | " | 2-$CH_3$ | HCl | $C_{21}H_{24}ClN$ | 325,87 | 225 | Cal. | 77,40 | 7,42 | 4,30 |
| | | | | | | | | | Tr. | 76,95 | 7,66 | 4,18 |
| 16 | " | " | " | 3-$CH_3$ | HCl+0,8% $H_2O$ | $C_{21}H_{24}ClN$ +0,8% $H_2O$ | 328,49 | 198 | Cal. | 76,78 | 7,45 | 4,27 |
| | | | | | | | | | Tr. | 76,52 | 7,69 | 4,22 |

TABLEAU I (Suite)

| Numéro de Code | 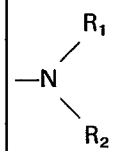 | n | R | $R_3$ | Forme | Formule brute | Poids molé-culaire | Point de fusion (°C) | ANALYSE ELEMENTAIRE | | | |
|---|---|---|---|---|---|---|---|---|---|---|---|---|
| | | | | | | | | | % | C | H | N |
| 17 | 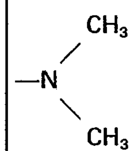 | 2 | H | 4-$CH_3$ | HCl | $C_{21}H_{24}ClN$ | 325,87 | 236 | Cal. | 77,40 | 7,42 | 4,30 |
| | | | | | | | | | Tr. | 77,43 | 7,67 | 4,41 |
| 18 | " | " | 6-Cl | H | " | $C_{20}H_{21}Cl_2N$ | 346,29 | 190 | Cal. | 69,36 | 6,11 | 4,05 |
| | | | | | | | | | Tr. | 69,07 | 6,44 | 4,06 |
| 19 | " | " | 7-Cl | " | HCl+1,9% $H_2O$ | $C_{20}H_{21}ClN$ +1,9% $H_2O$ | 353,03 | 190 | Cal. | 68,04 | 6,20 | 3,97 |
| | | | | | | | | | Tr. | 67,84 | 6,11 | 3,67 |
| 20 | " | " | 6-F | " | Oxalate | $C_{22}H_{22}FNO_4$ | 383,40 | 170 | Cal. | 68,91 | 5,78 | 3,65 |
| | | | | | | | | | Tr. | 68,60 | 5,66 | 3,87 |

TABLEAU I (Suite)

| Numéro de Code | | n | R | $R_3$ | Forme | Formule brute | Poids molé-culaire | Point de fusion (°C) | ANALYSE ELEMENTAIRE % | C | H | N |
|---|---|---|---|---|---|---|---|---|---|---|---|---|
| | 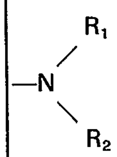 | | | | | | | | | | | |
| 21 | 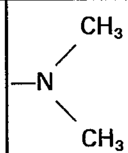 | 2 | 6-$CH_3$ | H | HCl+4,8% $H_2O$ | $C_{21}H_{24}NCl$ +4,8% $H_2O$ | 342,30 | Décomposition vers 100°C | Cal. | 73,68 | 7,60 | 4,09 |
| | | | | | | | | | Tr. | 73,57 | 7,47 | 4,18 |
| 22 | —$NHCH_3$ | " | H | 2-F | HCl | $C_{19}H_{19}ClFN$ | 315,81 | 190 | Cal. | 72,26 | 6,06 | 4,44 |
| | | | | | | | | | Tr. | 71,93 | 6,11 | 4,50 |
| 23 | " | 3 | " | H | " | $C_{21}H_{24}ClN$ | 325,87 | 208 | Cal. | 77,40 | 7,42 | 4,30 |
| | | | | | | | | | Tr. | 77,50 | 7,12 | 4,32 |

TABLEAU II

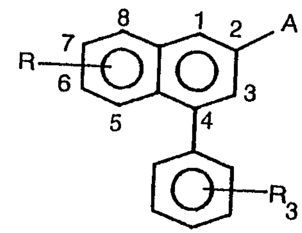

| Numéro de Code | R | $R_3$ | A | Formule brute | Poids moléculaire | Point de fusion (°C) | SPECTRE DE RMN (δ ppm =) |
|---|---|---|---|---|---|---|---|
| 1A | H | H | $CH_2OH$ | $C_{17}H_{14}O$ | 234,28 | 86 | ($CDCl_3$): 7,3 à 7,9 m (11 protons aromatiques) 4,8, s (—$CH_2O$—) 2,4, s (—OH) |
| 1B | „ | „ | $CH_2CH_2OH$ | $C_{18}H_{16}O$ | 248,31 | Huile | ($CDCl_3$): 7,2 à 8, m (11 protons aromatiques); 3,9, t (J=6Hz) (—$CH_2O$); 3,0, t, (J=6Hz) (—$CH_2$—); 1,8, s (OH) |
| 6A | „ | 2-F | $CH_2OH$ | $C_{17}H_{13}FO$ | 252,27 | 100 | ($CDCl_3$): 9, s et 7 à 7,7, m (10 protons aromatiques); 4,65, d (J=5Hz); ($CH_2O$); 2,6, t (J=5Hz) (OH) |
| 6B | „ | „ | $CH_2CH_2OH$ | $C_{18}H_{15}FO$ | 266,30 | Huile | ($CDCl_3$): 7,1 à 7,8, m (10 protons aromatiques); 3,9, t (J=6Hz) ($CH_2O$); 3,0, t (J=6Hz), ($CH_2O$); 1,7, s (OH) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 8A | H | 4-F | $CH_2OH$ | $C_{17}H_{13}FO$ | 252,27 | 75 | ($CDCl_3$): 6,9 à 7,8, m (10 protons aromatiques); 4,65, s ($C\underline{H}_2O$); 2,8, s ($O\underline{H}$) |
| 7A | 8-Cl | H | $CH_2OH$ | $C_{17}H_{13}ClO$ | 268,73 | Huile | ($CDCl_3$): 8,2, s (H aromatiques en 1); 6,9 à 7,8, m (9 protons aromatiques); 4,70, s ($C\underline{H}_2$—OH); 3,1, m ($O\underline{H}$) |
| 7B | " | " | $CH_2CH_2OH$ | $C_{18}H_{15}ClO$ | 282,76 | " | ($CDCl_3$): 8,1, s (H aromatique en 1); 6,9 à 7,8, m (9 protons aromatiques); 3,9, t, (J=6Hz) ($C\underline{H}_2O$); 3, t (J=6Hz) ($C\underline{H}_2$); 1,7, s ($O\underline{H}$) |
| 1C | H | H | COOH | $C_{17}H_{12}O_2$ | 248,27 | 253 | — |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 1D | H | H | COOEt | $C_{19}H_{16}O_2$ | 276,32 | Huile | (DMSO): 7,4 à 8,4, m, (11 protons aromatiques); 4,4, q (COOC<u>H</u>$_2$); 1,4, t (—C<u>H</u>$_3$) |
| 1E | " | " | $CH_2COOEt$ | $C_{20}H_{18}O_2$ | 290,34 | " | ($CDCl_3$): 7,2 à 7,9, m (11 protons aromatiques); 4,1, q (COOC<u>H</u>$_2$); 3,7, s (—C<u>H</u>$_2$—COO—); 1,2, t (—C<u>H</u>$_3$) |
| 1F | " | " | $CH_2CN$ | $C_{18}H_{13}N$ | 243,29 | " | ($CDCl_3$): 7,2 à 7,95, m (11 protons aromatiques); 3,9, s (C<u>H</u>$_2$—CN) |
| 6C | " | 2-F | COOH | $C_{17}H_{11}FO_2$ | 266,26 | 180 | ($CDCl_3$+DMSO): 11,6, m (COO<u>H</u>); 7 à 8, m (10H aromatiques) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 6D | H | 2-F | COOEt | $C_{19}H_{15}FO_2$ | 294,31 | Huile | ($CDCl_3$): 8,7, d et 7,1 à 8, m (10H aromatiques); 4,35, q (J=7Hz) (—$COOCH_2$); 1,35, t (J=7Hz) (—$CH_3$) |
| 6E | " | " | $CH_2COOEt$ | $C_{20}H_{17}FO_2$ | 308,34 | " | ($CDCl_3$): 7,1 à 7,8, m (10H aromatiques); 4,15, q (J=7Hz) ($COOCH_2$); 3,75, s ($CH_2$—COO); 1,2, t (J=7Hz) ($CH_3$) |
| 6F | " | " | $CH_2CN$ | $C_{18}H_{12}FN$ | 261,28 | " | ($CDCl_3$): 7,1 à 7,8, m (10H aromatiques); 3,8, s (—$CH_2$—CN) |
| 8C | " | 4-F | COOH | $C_{17}H_{11}FO_2$ | 266,26 | 260 | ($CDCl_3$): 12, m (COOH); 8,5, d et 7 à 8, m (10H aromatiques) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 8D | H | 4-F | COOEt | $C_{19}H_{15}FO_2$ | 294,31 | 76 | ($CDCl_3$): 8,7, d et 7 à 8, m (10H aromatiques); 4,5, q (J=7Hz) (COOC<u>H</u>$_2$); 1,4, t (J=7Hz) (C<u>H</u>$_3$) |
| 8E | " | " | $CH_2COOEt$ | $C_{20}H_{17}FO_2$ | 308,34 | Huile | ($CDCl_3$): 6,9 à 7,9, m (10H aromatiques); 4,1, q (J=7Hz) (COO—C<u>H</u>$_2$); 3,7, s (C<u>H</u>$_2$COO); 1,2, t (J=7Hz) (C<u>H</u>$_3$) |
| 8F | " | " | $CH_2CN$ | $C_{18}H_{12}FN$ | 261,28 | " | ($CDCl_3$): 6,9 à 7,9, m (10H aromatiques); 3,8, s (C<u>H</u>$_2$—CN) |
| 7C | 8-Cl | H | COOH | $C_{17}H_{11}ClO_2$ | 282,71 | 218 | (DMSO): 8,85, s (1H aromatique en 1); 7,1 à 7,9, m (9 protons aromatiques) |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 7D | 8-Cl | H | COOEt | $C_{19}H_{15}ClO_2$ | 310,77 | Huile | ($CDCl_3$+DMSO): 9,05, s (1H aromatique en 1); 7,2à8,m(9protonsaromatiques); 4,45, q (J=7Hz) (COOC$\underline{H}_2$); 1,4, t (J=7Hz) (C$\underline{H}_3$) |
| 7E | " | " | $CH_2COOEt$ | $C_{20}H_{17}ClO_2$ | 324,79 | " | ($CDCl_3$): 8,2, s (1H aromatique en 1); 7,1 à 7,8, m (9 protons aromatiques); 3,8, s (C$\underline{H}_2$—COO); 4,15, q (J=7Hz) (COOC$\underline{H}_2$); 1,2, t (J=7Hz) (C$\underline{H}_3$) |
| 7F | " | " | $CH_2CN$ | $C_{18}H_{12}ClN$ | 277,74 | " | ($CDCl_3$): 8,2, s (H aromatiques en 1); 6,9 à 7,8, m (9H aromatiques); 3,9, s (—C$\underline{H}_2$—CN) |
| 8B | H | 4-F | $CH_2CH_2OH$ | $C_{18}H_{15}FO$ | 266,30 | " | ($CDCl_3$): 6,9 à 7,9, m (10 protons aromatiques); 3,9, t (J=6Hz) (C$\underline{H}_2$O); 3, t (J=6Hz) (—C$\underline{H}_2$); 1,9, s (O$\underline{H}$) |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 14C | H | 3-F | COOH | $C_{17}H_{11}FO_2$ | 266,26 | 185 | — |
| 14A | " | " | $CH_2OH$ | $C_{17}H_{13}FO$ | 252,27 | 80 | 6,95 à 8, m (10 protons aromatiques)<br>4,8, s (—$CH_2$—O—)<br>2,3, s (—O$\underline{H}$) |
| 14D | " | " | COOEt | $C_{19}H_{15}FO_2$ | 294,31 | 100 | 6,95 à 8,7, m (10 protons aromatiques)<br>4,4, q (J=6Hz) (COOC$\underline{H_2}$—)<br>1,4, t (J=6Hz) (COO╱╲$CH_3$) |
| 14F | " | " | $CH_2CN$ | $C_{18}H_{12}FN$ | 261,28 | Huile | 6,9 à 7,95, m (10 protons aromatiques)<br>3,8, s (—C$\underline{H_2}$—CN) |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 14E | H | 3-F | $CH_2COOEt$ | $C_{20}H_{17}FO_2$ | 308,34 | Huile | 6,9 à 8, m (10 protons aromatiques)<br>4,1, q (J=6Hz) (COO—$C\underline{H}_2$—)<br>3,75, s (—$C\underline{H}_2$—COO—)<br>1,20, t (J=6Hz) (COO╱╲$C\underline{H}_3$) |
| 14B | " | " | $CH_2CH_2OH$ | $C_{18}H_{15}FO$ | 266,30 | " | 7 à 7,95, m (10 protons aromatiques)<br>3,95, (t (J=6Hz) (—$C\underline{H}_2$—O—)<br>3,0, t (J=6Hz) (—$C\underline{H}_2$—$CH_2$—O) 1,8, s (O$\underline{H}$) |
| 15C | " | 2-$CH_3$ | COOH | $C_{18}H_{14}O_2$ | 262,29 | 238 | — |
| 15A | " | " | $CH_2OH$ | $C_{18}H_{16}O$ | 248,31 | 82 | 7 à 8, m (10 protons aromatiques)<br>4,8, m ($C\underline{H}_2O$)<br>2,4, s (—$C\underline{H}_3$)<br>1,95, m (O$\underline{H}$) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 15F | H | 2-$CH_3$ | $CH_2CN$ | $C_{19}H_{15}N$ | 257,32 | Huile | 7 à 8, m (10 protons aromatiques)<br>3,8, m (—$C\underline{H}_2$—CN)<br>2,4, s (—$CH_3$) |
| 15E | " | " | $CH_2COOEt$ | $C_{21}H_{20}O_2$ | 304,37 | " | 7 à 8, m (10 protons aromatiques)<br>4,15, q (J=6Hz) (—$COOC\underline{H}_2$)<br>3,8, s ($C\underline{H}_2$—COO)<br>2,4, s (—$C\underline{H}_3$)<br>1,2, t (J=6Hz) (COO╱╲$C\underline{H}_3$) |
| 15B | " | " | $CH_2CH_2OH$ | $C_{19}H_{18}O$ | 262,33 | " | 7 à 8, m (10 protons aromatiques)<br>3,95, t (J=6Hz) (—$C\underline{H}_2$—O—)<br>3, t (J=6Hz) ($C\underline{H}_2$╱╲O)<br>2,4, s (—$C\underline{H}_3$) |
| 16B | " | 3-$CH_3$ | " | " | " | " | 7 à 8, m (10 protons aromatiques)<br>3,95, t (J=6Hz) (—$C\underline{H}_2$—O)<br>3, t (J=6Hz) ($C\underline{H}_2$╱╲O—)<br>2,4, s (—$C\underline{H}_3$)<br>1,6, s (O$\underline{H}$) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 16E | H | 3-$CH_3$ | $CH_2COOEt$ | $C_{21}H_{20}O_2$ | 304,37 | Huile | 6,9 à 8, m (10 protons aromatiques)<br>4,2, q (J=6Hz) (—COO—C<u>H</u>$_2$)<br>3,8, s (—C<u>H</u>$_2$—COO—)<br>2,4, s (—C<u>H</u>$_3$)<br>1,2, t (J=6Hz) (COO╱╲C<u>H</u>$_3$) |
| 16F | H | 3-$CH_3$ | $CH_2$—CN | $C_{19}H_{15}N$ | 257,32 | Huile | 7 à 7,95, m (10 protons aromatiques)<br>3,8, s (C<u>H</u>$_2$—CN)<br>2,4, s (—OC<u>H</u>$_3$) |
| 16A | " | " | $CH_2OH$ | $C_{18}H_{16}O$ | 248,31 | " | 7 à 8, m (10 protons aromatiques)<br>4,8, s (—C<u>H</u>$_2$—O—)<br>2,4, s (—C<u>H</u>$_3$)<br>2,0, s (—O<u>H</u>) |
| 16D | " | " | COOEt | $C_{20}H_{18}O_2$ | 290,34 | " | 7 à 8,6, m (10 protons aromatiques)<br>4,4, q (J=6Hz) (COOC<u>H</u>$_2$—)<br>2,4, s (—C<u>H</u>$_3$)<br>1,4, t (J=6Hz) (COO╱╲$CH_3$) |
| 16C | " | " | COOH | $C_{18}H_{14}O_2$ | 262,29 | 224 | 10, 1, m (COO<u>H</u>)<br>7 à 8,6, m (10 H aromatiques)<br>2,4, s (C<u>H</u>$_3$) |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 17B | H | 4-$CH_3$ | $CH_2CH_2OH$ | $C_{19}H_{18}O$ | 262,33 | Huile | 7 à 8, m (10 H aromatiques)<br>3,8, t (J=6Hz) (—$C\underline{H}_2$O—)<br>2,95, t (J=6Hz) (—$C\underline{H}_2$╱╲O—)<br>2,4, s (—$C\underline{H}_3$)<br>1,6, s (O$\underline{H}$) |
| 17E | " | " | $CH_2COOEt$ | $C_{21}H_{20}O_2$ | 304,37 | " | 7 à 8, m (10 H aromatiques)<br>4,1, q (J=6Hz) (—COO—$C\underline{H}_2$)<br>3,7, s (—$C\underline{H}_2$—COO)<br>2,4, s (—$C\underline{H}_3$)<br>1,2, t (J=6Hz) (COO╱╲$C\underline{H}_3$) |
| 17F | " | " | $CH_2$—CN | $C_{19}H_{15}N$ | 257,32 | " | 7 à 8, m (10 H aromatiques)<br>3,9, s (—$C\underline{H}_2$—CN)<br>2,4, s (—$C\underline{H}_3$) |
| 17A | " | " | $CH_2OH$ | $C_{18}H_{16}O$ | 248,31 | 84 | 7 à 8, m (10 H aromatiques)<br>4,8, s (—$C\underline{H}_2$O)<br>2,4, s (—$CH_3$)<br>2,2, s (—O$\underline{H}$) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 17C | H | 4-$CH_3$ | COOH | $C_{18}H_{14}O_2$ | 262,29 | 245 | 9, 3, m (COO$\underline{H}$)<br>7 à 8,5, m (10H aromatiques)<br>2,4, s (—C$\underline{H}_3$) |
| 18B | 6-Cl | H | $CH_2CH_2OH$ | $C_{18}H_{15}ClO$ | 282,76 | 120 | 6,7 à 7,8, m (10H aromatiques)<br>3,9, t (J=6Hz), (—C$\underline{H}_2$O—)<br>2,9, t (J=6Hz) (C$\underline{H}_2$/\O—)<br>2,1, s (O$\underline{H}$) |
| 18E | " | " | $CH_2COOEt$ | $C_{20}H_{17}ClO_2$ | 324,79 | Huile | — |
| 18F | " | " | $CH_2CN$ | $C_{18}H_{12}ClN$ | 277,74 | " | — |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 18A | 6-Cl | H | $CH_2OH$ | $C_{17}H_{13}ClO$ | 268,73 | Pâte | 6,8 à 7,8, m (10H aromatiques)<br>4,7, s ($-C\underline{H}_2O-$)<br>2,4, m ($O\underline{H}$) |
| 18D | " | " | COOEt | $C_{19}H_{15}ClO_2$ | 310,77 | Huile | 6,7 à 8,8, m (10H aromatiques)<br>4,4, q (J=6Hz) ($COOC\underline{H}_2$)<br>1,4, t (J=6Hz) (COO╱╲$C\underline{H}_3$) |
| 18C | " | " | COOH | $C_{17}H_{11}ClO_2$ | 282,71 | >250 | — |
| 19B | 7-Cl | " | $CH_2CH_2OH$ | $C_{18}H_{15}ClO$ | 282,76 | Huile | 7,1 à 7,8, m (10H aromatiques)<br>3,9, t (J=6Hz) ($-C\underline{H}_2O$)<br>3,0, t (J=6Hz) ($C\underline{H}_2$╱╲O—)<br>1,6, m ($O\underline{H}$) |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 19E | 7-Cl | H | $CH_2COOEt$ | $C_{20}H_{17}ClO_2$ | 324,79 | Huile | 7 à 7,8, m (10H aromatiques)<br>4,1, q (J=6Hz) (COO—C$\underline{H}_2$)<br>3,75, s (—C$\underline{H}_2$—COO—)<br>1,2, t (J=6Hz) (COO╱╲C$\underline{H}_3$) |
| 19F | " | " | $CH_2$—CN | $C_{18}H_{12}ClN$ | 277,74 | " | 7 à 7,9, m (10H aromatiques)<br>3,9, s (C$\underline{H}_2$—CN) |
| 19A | " | " | $CH_2OH$ | $C_{17}H_{13}ClO$ | 268,73 | Pâte | 7,1 à 7,8, m (10H aromatiques)<br>4,7, s (—C$\underline{H}_2$O)<br>2,95, s (O$\underline{H}$) |
| 19D | " | " | COOEt | $C_{19}H_{15}ClO_2$ | 310,77 | Huile | 6,9 à 8,4, m (10H aromatiques)<br>4,4, q (—COOC$\underline{H}_2$—) (J=6Hz)<br>1,4, t (J=6Hz) (COO╱╲C$\underline{H}_3$) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 19C | 7-Cl | H | COOH | $C_{17}H_{11}ClO_2$ | 282,71 | 155 | — |
| 20B | 6-F | " | $CH_2CH_2OH$ | $C_{18}H_{15}FO$ | 266,30 | Huile | 7 à 7,9, m (10H aromatiques)<br>3,9, t (J=6Hz), (—C<u>H</u>$_2$—O—)<br>3,0, t (J=6Hz) (—$CH_2$/\O—)<br>1,8, m (O<u>H</u>) |
| 20E | " | " | $CH_2COOEt$ | $C_{20}H_{17}FO_2$ | 308,33 | " | 7 à 7,9, m (10H aromatiques)<br>4,1, q (J=6Hz) (—COO—C<u>H</u>$_2$—)<br>3,7, s (—C<u>H</u>$_2$—COO—)<br>1,2, t (J=6Hz) (COO/\C<u>H</u>$_3$) |
| 20F | " | " | $CH_2CN$ | $C_{18}H_{12}FN$ | 261,28 | " | 7,1 à 8, m (10H aromatiques)<br>3,9, s (—C<u>H</u>$_2$CN) |

TABLEAU II (Suite)

| Numéro de Code | R | $R_3$ | A | Formule brute | Poids moléculaire | Point de fusion (°C) | SPECTRE DE RMN (δ ppm =) |
|---|---|---|---|---|---|---|---|
| 20A | 6-F | H | $CH_2OH$ | $C_{17}H_{13}FO$ | 252,27 | 88 | 6,9 à 7,8, m (10H aromatiques)<br>4,65, s (—C<u>H</u>$_2$O)<br>2,9, s (—O<u>H</u>) |
| 20D | " | " | COOEt | $C_{19}H_{15}FO_2$ | 294,31 | Huile | 7,1 à 8,5, m (10H aromatiques)<br>4,4, q (J=6Hz) (COOC<u>H</u>$_2$—)<br>1,4, t (J=6Hz) (COO—$CH_2$—C<u>H</u>$_3$) |
| 20C | " | " | COOH | $C_{17}H_{11}FO_2$ | 266,26 | 260 | — |
| 21B | 6-$CH_3$ | " | $CH_2CH_2OH$ | $C_{19}H_{18}O$ | 262,33 | Huile | 7 à 7,7, m (10H aromatiques)<br>3,9, t (J=6Hz) (—C<u>H</u>$_2$O—)<br>2,95, t (J=6Hz) (—C<u>H</u>$_2$╱╲O—)<br>2,4, s (—C<u>H</u>$_3$) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 21E | 6-$CH_3$ | H | $CH_2COOEt$ | $C_{21}H_{20}O_2$ | 304,37 | Huile | 7 à 7,7, m (10H aromatiques)<br>4,1, q (J=6Hz) (COOC$\underline{H}_2$—)<br>3,7, s (C$\underline{H}_2$COO)<br>2,4, s (—C$\underline{H}_3$)<br>1,2, t (J=6Hz) (COO╱╲C$\underline{H}_3$) |
| 21A | " | " | —$CH_2OH$ | $C_{18}H_{16}O$ | 248,31 | 128 | 7,2 à 7,8, m (10H aromatiques)<br>4,8, s (—C$\underline{H}_2$O)<br>2,4, s (—C$\underline{H}_3$)<br>1,8, m (—O$\underline{H}$) |
| 21D | " | " | —COOEt | $C_{20}H_{18}O_2$ | 290,34 | Huile | 6,9 à 8,5, m (10H aromatiques)<br>4,4, q (J=6Hz) (COOC$\underline{H}_2$)<br>2,4, s (C$\underline{H}_3$)<br>1,4, t (J=6Hz) (COO╱╲C$\underline{H}_3$) |
| 21C | " | " | —COOH | $C_{18}H_{14}O_2$ | 262,29 | >300 | 7,3 à 8,7, m (10H aromatiques)<br>2,4, s (—C$\underline{H}_3$) |

TABLEAU II (Suite)

| Numéro de Code | R | $R_3$ | A | Formule brute | Poids molé-culaire | Point de fusion (°C) | SPECTRE DE RMN (δ ppm =) |
|---|---|---|---|---|---|---|---|
| 1G | H | H | $CH_2CH_2CH_2OH$ | $C_{19}H_{18}O$ | 262,00 | Huile | 7,1 à 7,9, m (11H aromatiques)<br>3,6, t (J=6Hz) ($C\underline{H}_2$—O—)<br>2,8, t (J=6Hz) ($C\underline{H}_2$)⁄⁄O—)<br>1,9, m (⁄╲$C\underline{H}_2$⁄╲O—)<br>2,4, s (—O$\underline{H}$) |

Les composés selon l'invention ont été étudiés chez l'animal de laboratoire et ont montré des propriétés pharmacologiques, notamment des activités dans le domaine du système nerveux central et en particulier des propriétés analgésiques et/ou antidépressives.

L'activité analgésique a été mise en évidence chez la souris (par administration i.p. des composés selon l'invention) en utilisant le test de la phénylbenzoquinone selon le protocole décrit par E. SIEGMUND dans Proced. Soc. Exp. Biol. and Med. *95*, 729, (1957).

L'activité antidépressive a quant à elle été mise en évidence chez la souris par le test de l'antagonisme vis-à-vis du ptosis observé une heure après une injection intraveineuse (2 mg/kg) de réserpine chez la souris selon le protocole décrit par C. GOURET et J. THOMAS, dans J. Pharmacol. (Paris), *4*, 401, (1973).

Pour illustrer l'invention, on a rassemblé dans le tableau III ci-après les résultats obtenus dans cez deux tests avec quelques composés selon l'invention. Ce tableau donne en outre la toxicité aiguë (DL 50) des composés testés.

TABLEAU III

| Numéro de Code | DL 50 souris (mg/kg/i.p.) | Propriétés analgésiques DE 50 (mg/kg/i.p.) | Propriétés antidépressives DE 50 (mg/kp/i.p.) |
|---|---|---|---|
| 1 | 140 | 12,5 | 10 |
| 2 | 70 | 4,5 | 5,3 |
| 3 | 47 | 6,2 | 3,2 |
| 4 | 83 | 3,2 | 0,6 |
| 5 | 165 | 6 | >10 |
| 6 | >100 | — | 0,6 |
| 7 | 90 | 2,8 | — |
| 8 | 115 | 3 | 2,5 |
| 9 | 58 | 2,2 | 3 |
| 10 | 90 | 2,4 | 0,6 |
| 11 | 75 | 10 | — |
| 12 | 82 | 7,4 | — |
| 13 | 260 | 24 | 18 |
| 14 | 93 | 2,8 | 0,8 |
| 15 | 90 | 5 | 5 |
| 16 | 140 | 4,4 | 17 |
| 17 | 130 | 3,5 | 3,5 |
| 18 | 120 | 4,8 | 9 |
| 19 | 110 | 5 | 8,7 |
| 20 | 90 | 6,2 | 3,7 |
| 21 | 65 | 6,2 | 10 |
| 22 | 77 | 1 | 0,21 |
| 23 | 71 | 2,4 | 1 |

L'écart entre les doses toxiques et les doses actives permet l'emploi en thérapeutique des composés selon l'invention.

La présente invention a ainsi également pour objet l'application, à titre de médicaments, des dérivés de formule (I) et leurs sels d'addition d'acide pharmaceutiquement acceptables, ces médicaments trouvant leur emploi dans le traitement des troubles du système nerveux central, notamment comme antidépresseurs et/ou analgésiques.

L'invention a enfin pour objet les compositions pharmaceutiques qui renferment l'un au moins des médicaments définis ci-dessus en association avec un support approprié pharmaceutiquement acceptable.

Ces compositions seront administrées:

— par voie orale, sous forme de comprimés, dragées, gélules, etc ..., à des doses pouvant aller jusqu'à 400 mg de principe actif/jour en 3 ou 4 prises;

— par voie intraveineuse, sous forme d'ampoules injectables à des doses pouvant aller jusqu'à 100 mg de principe actif/jour en 3 ou 4 prises;

— par voie intramusculaire, sous forme d'ampoules injectables à des doses pouvant aller jusqu'à 100 mg de principe actif/jour en 3 ou 4 prises; ou

— sous forme de suppositoires à des doses pouvant aller jusqu'à 200 mg de principe actif/jour en 3 ou 4 prises.

## Revendications

1. Nouveaux dérivés aminoalkyl naphtaléniques répondant à la formule:

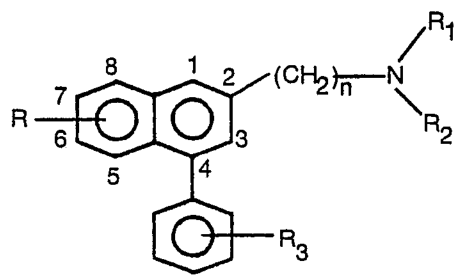

(I)

dans laquelle:

$R_1$ et $R_2$ sont identiques ou différents et représentent un atome d'hydrogène ou un groupe alkyle comportant de 1 à 4 atomes de carbone ou forment conjointement avec l'atome d'azote auquel ils sont liés, un radical hétérocyclique choisi parmi les suivants: pyrrolidino, pipéridino, hexaméthylèneimino, morpholino, pipérazino, méthyl-4 pipérazino;

$n = 1, 2$ ou 3;

R représente un atome d'hydrogène ou d'halogène ou un groupe méthyle; et

$R_3$ représente un atome d'hydrogène ou d'halogéne ou un groupe méthyle ou méthoxy;

ainsi que leurs sels d'addition d'acide organique ou minéral.

2. Dérivé et sel selon la revendication 1, caractérisés en ce que $NR_1R_2$ représente $NH_2$, $n = 2$, $R = H$ et $R_3 = H$.

3. Dérivés et sels selon la revendication 1, caractérisés en ce que $NR_1R_2$ représente $NHCH_3$ et l'ensemble (n, R, $R_3$) prend l'une quelconque des valeurs suivantes: (1, H, H), (2, H, H), (2, H, ortho-F).

4. Dérivés et sels selon la revendication 1, caractérisés en ce que $NR_1R_2$ représente $N(CH_1)_2$, et l'ensemble (n, R, $R_3$) prend l'une quelconque des valeurs suivantes: (1, H, H), (2, H, H), 2, H, ortho-F), (2,8-Cl, H), (2, H, para-F), (2, H, méta-F), (2, H, ortho-$CH_3$), (2, H, méta-$CH_3$), (2, H, para-$CH_3$), (2,6-Cl, H), (2, 7-Cl, H), (2,6-F, H), (2,6-$CH_3$, H), (3, H, H).

5. Dérivés et sels selon la revendication 1, caractérisés en ce que $R = H$, $R_3 = H$, $n = 1$ ou 2 et $NR_1R_2$ représente

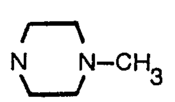

6. Dérivés et sels selon la revendication 1, caractérisés en ce que $n = 2$, $R = H$, $R_3 = H$ et $NR_1R_2$ représente le radical pyrrolidino, pipéridino ou morpholino.

7. Médicament notamment à activité analgésique et/ou antidépressive, caractérisé en ce qu'il est constitué par l'un quelconque des dérivés et sels pharmaceutiquement acceptables selon l'une des revendications 1 à 6.

8. Composition pharmaceutique, caractérisée en ce qu'elle comprend au moins l'un des médicaments selon la revendication 7 en association avec un support pharmaceutiquement acceptable.

9. Procédé de préparation des dérivés de formule (I) et de leurs sels selon les revendications 1 à 6, caractérisé en ce qu'il consiste à condenser les amines de formule:

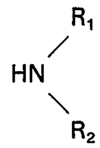

(II)

dans laquelle $R_1$ et $R_2$ ont la même signification que dans la revendication 1, respectivement avec les composés de formule:

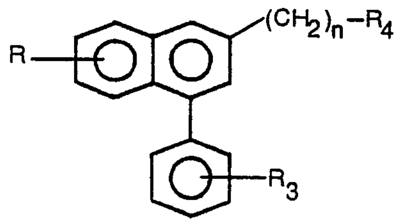

(III)

dans laquelle n, R et $R_3$ ont la même signification que dans la revendication 1 et $R_4$ représente un groupement nucléophile bon partant; puis à éventuellement salifier les dérivés obtenus.

10. Procédé selon la revendication 9, caractérisé en ce que le groupement nucléophile est constitué par un atome d'halogène ou par le groupe méthylsulfonyloxy ou p-tolylsulfonyloxy.

11. Procédé selon la revendication 9 ou 10, caractérisé en ce que la condensation est effectuée dans un solvant aprotique.

12. Intermédiaires de synthèse caractérisés en ce qu'il répondent:

— à la formule:

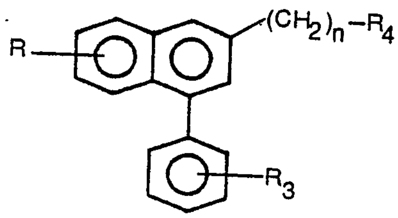

(III)

où n, R et $R_3$ ont les mêmes significations que dans la revendication 1 et $R_4$ représente le groupement méthylsulfonyloxy;

— à la formule:

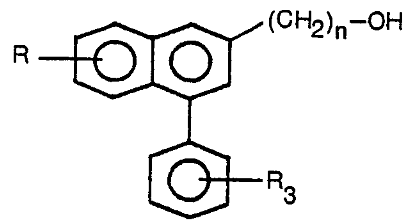

(IV)

où n, R et $R_3$ ont les mêmes significations que dans la revendication 1; ou

— à la formule:

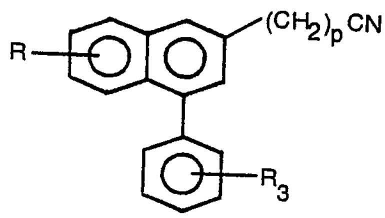

(X)

où R et $R_3$ ont les mêmes significations que dans la revendication 1 et p = 1 ou 2.

**Patentansprüche**

1. Neue Aminoalkylnaphthalinderivate, die der Formel entsprechen:

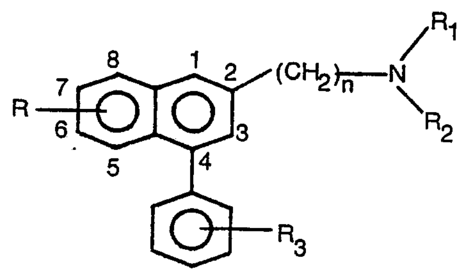

(I)

worin bedeuten:

$R_1$ und $R_2$, die gleich oder verschieden sind, ein Wasserstoffatom oder eine Alkylgruppe mit 1 bis 4 Kohlenstoffatomen oder die gemeinsam zusammen mit dem Stickstoffatom, an das sie gebunden sind, einen heterocyclischen Rest bilden, der ausgewählt wird aus Pyrrolidino, Piperidino, Hexamethylenimino, Morpholino, Piperazino, 4-Methyl-piperazino;

n = 1, 2 oder 3;

R ein Wasserstoffatom oder ein Halogenatom oder eine Methylgruppe; und

$R_3$ ein Wasserstoffatom oder ein Halogenatom oder eine Methyl- oder Methoxygruppe;

sowie ihre Säureadditionssalze mit organischen oder Mineralsäuren.

2. Derivat und Salz nach Anspruch 1, dadurch gekennzeichnet, daß $NR_1R_2$ $NH_2$ darstellt, n = 2, R = H und $R_3$ = H.

3. Derivate und Salze nach Anspruch 1, dadurch gekennzeichnet, daß $NR_1R_2$ $NHCH_3$ darstellt und die übrigen Symbole (n, R, $R_3$) irgendeine der folgenden Bedeutungen haben (1, H, H), (2, H, H), (2, H, o—F).

4. Derivate und Salze nach Anspruch 1, dadurch gekennzeichnet, daß $NR_1R_2$ $N(CH_3)_2$ darstellt und die übrigen Symbole (n, R, $R_3$) irgendeine der folgenden Bedeutungen haben: (1, H, H), (2, H, H), 2, H, o-F), (2,8-Cl, H), (2, H, p-F), (2, H, m-F), (2, H, o-$CH_3$), (2, H, m-$CH_3$), (2, H, p-$CH_3$), (2,6-Cl, H), (2, 7-Cl, H), (2,6-F, H), (2,6-$CH_3$, H), (3, H, H).

5. Derivate und Salze nach Anspruch 1, dadurch gekennzeichnet, daß R = H, $R_3$ = H, n = 1 oder 2 und $NR_1R_2$

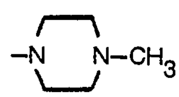

darstellt.

6. Derivate und Salze nach Anspruch 1, dadurch gekennzeichnet, daß n = 2, R = H, $R_3$ = H und $NR_1R_2$ den Pyrrolidino-, Piperidino- oder Morpholinorest darstellt.

7. Arzneimittel insbesondere mit analgetischer und/oder antidepressiver Aktivität, dadurch gekennzeichnet, daß es besteht aus irgendeinem der pharmazeutisch verträglichen Derivate und Salze nach einem der Ansprüche 1 bis 6.

8. Pharmazeutische Zusammensetzung, dadurch gekennzeichnet, daß sie umfaßt mindestens eines der Arzneimittel nach Anspruch 7 in Assoziation mit einem pharmazeutisch verträglichen Träger.

9. Verfahren zur Herstellung der Derivate der Formel (I) und ihrer Salz nach den Ansprüchen 1 bis 6, dadurch gekennzeichnet, daß es darin besteht, daß man kondensiert die Amine der Formel

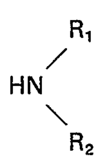

(II)

worin $R_1$ und $R_2$ die gleiche Bedeutung wie im Anspruch 1 haben, jeweils mit Verbindungen der Formel

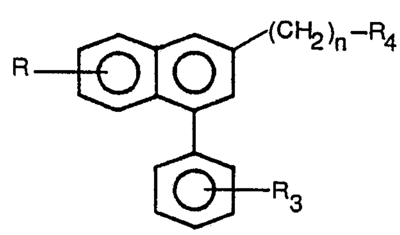

(III)

worin n, R und $R_3$ die gleiche Bedeutung wie in Anspruch 1 haben und $R_4$ eine gut abspaltbare nukleophile Gruppe darstellt, und dann ggf. die erhaltenen Derivate in ein Salz überführt.

10. Verfahren nach Anspruch 9, dadurch gekennzeichnet, daß die nukleophile Gruppe aus einem Wasserstoffatom oder aus einer Methylsulfonyloxy- oder p-Tolysulfonyloxygruppe besteht.

11. Verfahren nach Anspruch 9 oder 10, dadurch gekennzeichnet, daß die Kondensation in einem aprotischen Lösungmittel durchgeführt wird.

12. Synthese-Zwischenprodukte, dadurch gekennzeichnet, daß sie eine der folgenden Formeln haben:

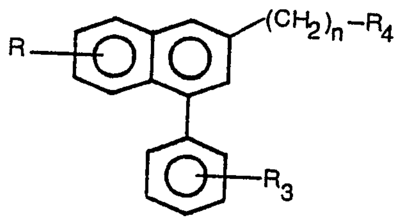

(III)

worin n, R und $R_3$ die gleichen Bedeutungen wie im Anspruch 1 haben und $R_4$ die Methylsulfonyloxygruppe darstellt;

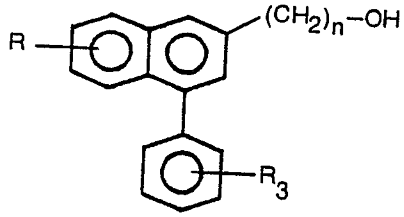

(IV)

worin n, R und $R_3$ die gleichen Bedeutungen wie im Anspruch 1 haben; oder

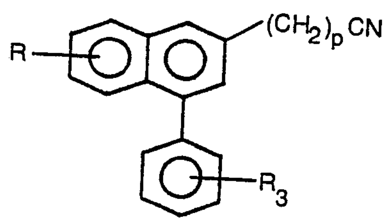

(X)

worin R und $R_3$ die gleichen Bedeutungen wie im Anspruch 1 haben und p = 1 oder 2.

**Claims**

1. New naphthalenic aminoalkyl derivatives corresponding to the formula:

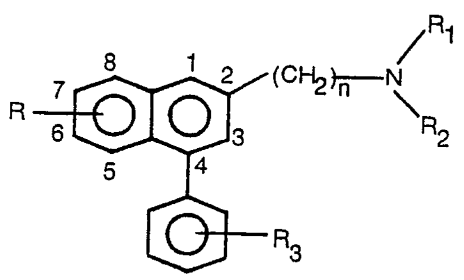

(I)

in which:

$R_1$ and $R_2$ are identical or different and represent a hydrogen atom or an alkyl group comprising 1 to 4 carbon atoms or form, conjointly with the nitrogen atom to which they are linked, a heterocyclic radical chosen from the following: pyrrolidino, piperidino, hexamethyleneimino, morpholino, piperazino, 4-methyl piperazino;

n = 1, 2 or 3;

R represents a hydrogen or halogen atom or a methyl group; and

$R_3$ represents a hydrogen or halogen atom or a methyl or methoxy group, as well as the organic or mineral acid addition salts thereof.

2. The derivatives and salts as claimed in claim 1, characterized in that $NR_1R_2$ represents $NH_2$, n = 2, R = H and $R_3$ = H.

3. The derivatives and salts as claimed in claim 1, characterized in that $NR_1R_2$ represents $NHCH_3$, and the set $(n,R,R_3)$ takes any one of the following values: (1,H,H), (2,H,H), (2,H,o-F).

4. The derivatives and salts as claimed in claim 1, characterized in that $NR_1R_2$ represents $N(CH_3)_2$ and the set $(n,R,R_3)$ takes any one of the following values: (1, H, H), (2, H, H), 2, H, o-F), (2,8-Cl, H), (2, H, p-F), (2, H, m-F), (2, H, o-$CH_3$), (2, H, m-$CH_3$), (2, H, p-$CH_3$), (2,6-Cl, H), (2, 7-Cl, H), (2,6-F, H), (2,6-$CH_3$, H), (3, H, H).

5. The derivatives and salts as claimed in claim 1, characterized in that R = H, $R_3$ = H, n = 1 or 2 and $NR_1R_2$ represents

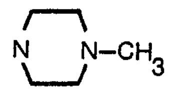

6. The derivatives and salts as claimed in claim 1, characterized in that n = 2, R = H, $R_3$ = H and $NR_1R_2$ represents the pyrrolidino, piperidino or morpholino radical.

7. A drug more especially with analgesic and/or antidepressive activity, characterized in that it is formed by any one of the derivatives and pharmaceutically acceptable salts as claimed in any one of claims 1 to 6.

8. A pharmaceutical composition, characterized in that it comprises at least one of the drugs as claimed in claim 7 in association with a pharmaceutically acceptable carrier.

9. A process for preparing the derivatives of formula (I) and the salts thereof as claimed in claims 1 to 6, characterized in that it consists in condensing the amines of formula:

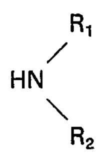

(II)

in which $R_1$ and $R_2$ have the same meaning as in claim 1, respectively with the compounds of formula:

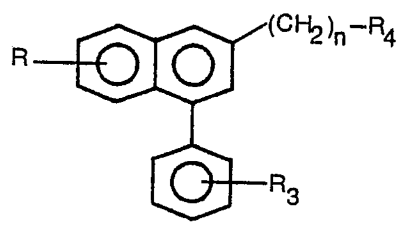

(III)

in which n, R and $R_3$ have the same meaning as in claim 1 and $R_4$ represents a good leaving nucleophilic group; then possibly in salifying the derivatives obtained.

10. The process as claimed in claim 9, characterized in that the nucleophilic group is formed by a halogen atom or by the methylsulfonyloxy or p-tolylsulfonyloxy group.

11. The process as claimed in claim 9 or 10, characterized in that the condensation is carried out in an aprotic solvent.

12. Synthesis intermediates, characterized in that they correspond:

— to the formula:

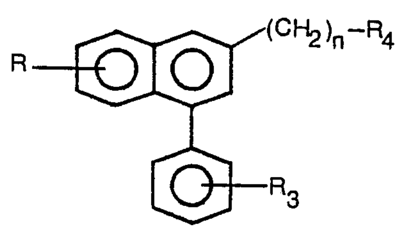

(III)

where n, R and $R_3$ have the same meanings as in claim 1 and $R_4$ represents the methylsulfonyloxy group;

— to the formula:

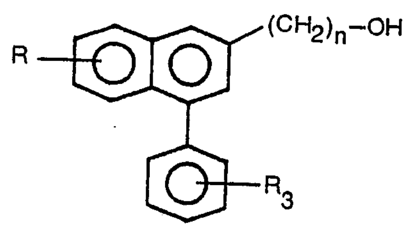

(IV)

in which n, R and $R_3$ have the same meanings as in claim 1; or
— to the formula:

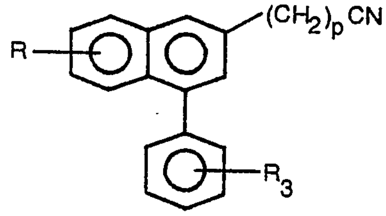

(X)

wherein R and $R_3$ have the same meanings as in claim 1 and p = 1 or 2.